# FASCICULE DE BREVET EUROPEEN

(11) **EP 1 112 261 B1**
(45) Date de publication et mention de la délivrance du brevet: **10.04.2002**
(21) Numéro de dépôt: 99934784.2
(22) Date de dépôt: 26.07.1999
(51) Int. Cl.: C07D 307/85, C07D 405/04

(54) **DERIVE D'ESTER D'ACIDE 2-ETHYL-2,3-DIHYDROBENZOFURANE-CARBOXYLIQUE, PROCEDE DE PREPARATION ET UTILISATION POUR LA PREPARATION DE DERIVES DE L'EFAROXAN**
2-ETHYL-2,3-DIHYDROBENZOFURAN-CARBONSÄURE-ESTER-DERIVAT, HERSTELLUNGSVERFAHREN UND VERWENDUNG ZUR HERSTELLUNG VON EFAROXAN-DERIVATEN
2-ETHYL-2,3-DIHYDROBENZOFURAN-CARBOXYLIC ACID ESTER DERIVATIVE, PREPARATION METHOD AND USE FOR PREPARING EFAROXAN DERIVATIVES

(30) Priorité: 10.09.1998 FR 9811303
(43) Date de publication de la demande: 04.07.2001
(73) Titulaire: PIERRE FABRE SANTE S.A., 92100 Boulogne (FR)
(72) Inventeur: MIOSKOWSKI, Charles, F-67200 Strasbourg (FR); GOUVERNEUR, Véronique, F-67000 Strasbourg (FR); COUTURE, Karine, F-69350 La Mulatière (FR); LESIMPLE, Patrick, F-81600 Brens (FR); AUTRET, Jean-Marie, F-81600 Gaillac (FR)
(74) Mandataire: Doat, Jean-Pierre
(86) Numéro de dépôt international: FR9901848
(87) Numéro de publication internationale: WO0015624

(56) Documents cités:
- EP-A- 0 071 368
- EP-A- 0 310 745
- WO-A-92/05171
- WO-A-96/35682
- HORAGUCHI T. ET AL.: "Photocyclization reactions." JOURNAL OF HETEROCYCLIC CHEMISTRY., vol. 28, no. 5, 1991, pages 1273-1280, XP002102303 PROVO US

## Description

La présente invention concerne un dérivé d'ester d'acide 2-éthyl-2,3-dihydro-benzofurane-carboxylique, son procédé de préparation et son utilisation pour la préparation d'un dérivé du 2-(2-(2-éthyl-2,3-dihydrobenzofuranyl))-2-imidazoline, en particulier de l'Efaroxan.

Les dérivés du 2-(2-(2-éthyl-2,3-dihydro-benzofuranyl))-2-imidazoline, en particulier de I'Efaroxan, sont décrits dans la demande de brevet EP-A-71 368 publiée le 09/02/1983. Ce document décrit également leur activité antagoniste sur des récepteurs alpha 2-adrénergiques (récepteurs présynatiques ayant pour rôle d'inhiber la libération de noradréaline afin de maintenir la concentration du neuro-transmetteur constante dans la jonction synaptique).

Les dérivés de la famille de l'Efaroxan sont efficaces pour augmenter la concentration de noradrénaline sur les sites postsynaptiques et sont connus pour le traitement de la dépression et de la migraine.

Les dérivés de la famille de l'Efaroxan sont également décrits dans la demande de brevet WO 95/01791 pour le traitement de la maladie d'Alzheimer, dans la demande de brevet WO 95/00145 pour le traitement de la maladie de Parkinson, et dans la demande de brevet WO 92/05171 comme agent bloqueur de canaux potassium, pour traiter le diabète.

La présente invention concerne un nouveau procédé de préparation des dérivés du 2-(2-(2-éthyl-2,3-dihydro-benzofuranyl))-2-imidazoline, de formule générale I

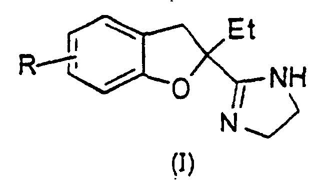

Dans l'ensemble du texte de la présente demande, R représente un atome d'hydrogène, un atome d'halogène, un radical alkyle inférieur, alkoxy inférieur ou hydroxy.

La synthèse des dérivés de formule générale I, plus particulièrement décrite dans la demande de brevet EP-A-71 368, suit le schéma réactionnel suivant : 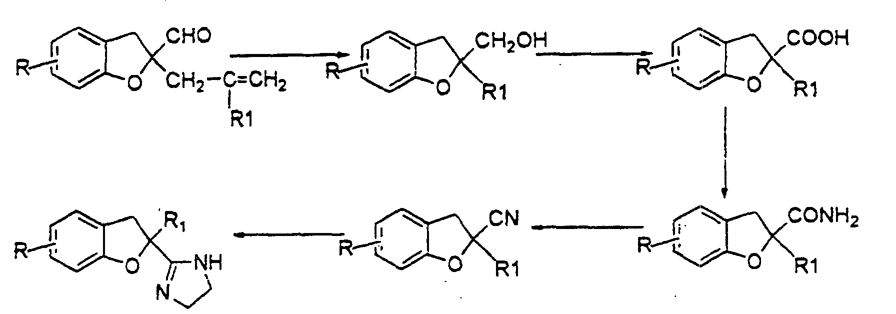 dans lequel R₁ est un hydrogène ou un radical alkyle en C₁-C₆ et R a la signification donnée précédemment.

L'étape-clé de la synthèse est la transformation du nitrile en imidazoline : le nitrile est dissous dans l'éthanol ou le méthanol puis traité successivement par un alkoxyde de sodium, par l'acide chlorhydrique puis par au moins un équivalent molaire d'éthylènediamine. L'imidazoline est obtenu avec un rendement molaire moyen de 90 %. L'inconvénient de cette synthèse qui comporte 8 étapes est son mauvais rendement global, inférieur à 10 % en mole.

CHAPLEO et AL. (J. Med. Chem., 1984, 27(5), pp. 570-576) proposent pour leur part la synthèse des dérivés de formule générale I tout en décrivant leur activité inhibitrice des alpha 2-adrénorécepteurs. L'intermédiaire clé de cette synthèse est l'acide 2-alkyl-2,3-dihydro-benzofurane-carboxylique, obtenu à partir d'un dérivé d'aldéhyde salicylique correspondant suivant le schéma suivant : 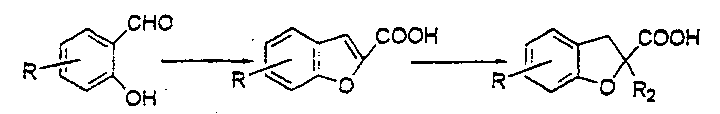 dans lequel R₂ est un hydrogène ou un radical méthyle, éthyle, propyle ou pentyle.
L'imidazoline est obtenu à partir de l'intermédiaire acide carboxylique selon le même schéma que celui décrit dans la demande de brevet EP-A-71 368.

Bien que cette synthèse ne comporte que six étapes et présente l'avantage de partir de l'aldéhyde salicylique, produit commercial peu coûteux, le rendement global ne dépasse pas les 10 % en mole.

EDWARDS et AL. (J. Heterocyclic Chem., 1987, 24, pp 495-496) décrivent la synthèse des dérivés de formule générale I en proposant un nouveau chemin pour passer de l'aldéhyde salicylique à l'acide 2,3-dihydrobenzofurane-carboxylique substitué en position 2 par un groupement R₃ représentant un hydrogène, un radical éthyle ou phényle. Le schéma réactionnel est le suivant :

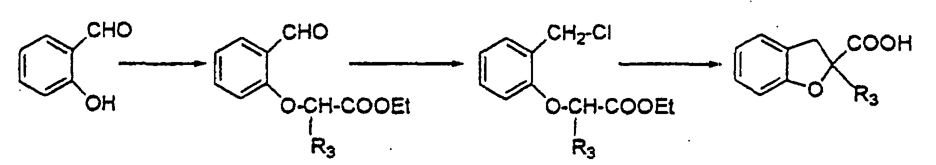

Cette synthèse, comportant 8 étapes, présente un meilleur rendement global, égal à 43 % lorsque R₃ = H.

La présente invention concerne un nouveau procédé de préparation des dérivés imidazoline de formule générale I, comportant 4 ou 5 étapes, sans passer par l'intermédiaire acide 2-alkyl-2,3-dihydrobenzofurane-carboxylique, et dont le rendement global est supérieur à 25 % en mole.

L'intermédiaire clé du procédé de préparation selon l'invention est le dérivé de formule générale de formule générale F 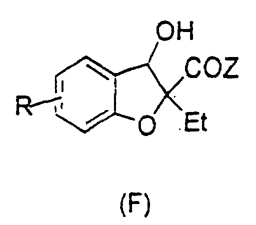 dans laquelle Z représente un groupement alkoxy en C₁-C₃, de préférence éthoxy, ou un groupement amino.

La présente invention concerne le dérivé de formule générale F à l'exception des dérivés dans lesquels R représente un atome d'hydrogène et Z un groupement éthoxy, ainsi que son procédé de préparation à partir du dérivé de l'aldéhyde salicylique correspondant de formule générale VI

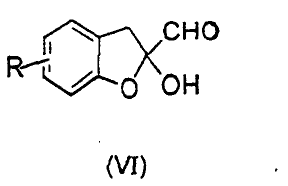

Selon le mode préféré de réalisation de l'invention, un équivalent du dérivé d'aldéhyde salicylique de formule générale VI est mis en présence d'un équivalent de 2-bromobutanoate d'éthyle et de deux équivalents de base, par exemple le carbonate de potassium, le tout dans un solvant polaire aprotique anhydre, par exemple le diméthylformamide. La réaction peut être catalysée par l'iodure de-sodium. Le mélange est agité et porté au reflux du solvant pendant environ 9 heures. Le dérivé ester obtenu, 2-éthylcarboxylate-2-éthyl-3-hydroxy-2,3-dihydrobenzofurane, de formule générale V 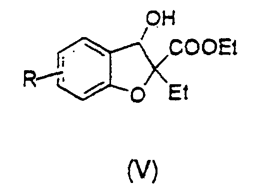 est purifié par des méthodes classiques, par exemple par chromatographie sur gel de silice.

La présente invention concerne le procédé de préparation du dérivé de formule générale F avec Z = amino, qui consiste à faire subir au dérivé ester de formule générale V correspondant, susceptible d'être obtenu par le procédé de préparation selon l'invention, une réaction d'aminolyse.

Le dérivé ester de formule générale V est transformé par le procédé de préparation selon l'invention, en un dérivé 2-carboxamide-2-éthyl-3-hydroxy-2,3-dihydrobenzofurane de formule générale IV

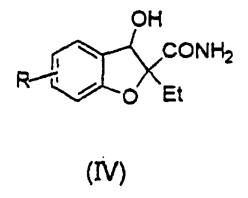

L'aminolyse peut être conduite selon deux voies : par une solution ammoniaquale ou par l'ammoniac.

L'aminolyse par une solution ammoniaquale consiste à dissoudre le dérivé ester de formule générale V dans un solvant polaire aprotique, par exemple le dioxane, puis à ajouter un excès d'une solution d'hydroxyde d'ammonium. Le mélange est agité à une température comprise entre 20 et 50°C pendant 60 à 140 heures.

L'aminolyse par ('ammoniac consiste à dissoudre le dérivé ester de formule générale V dans un solvant polaire protique, par exemple un alcool aliphatique en C₁ - C₃. de préférence le méthanol. La solution est saturée en ammoniac gazeux puis agitée à une température comprise entre 15 et 30°C pendant 5 jours.

Le dérivé de formule générale IV, obtenu par l'une quelconque des deux voies, est purifié par des méthodes classiques, par exemple par extraction au solvant chloré puis par chromatographie sur gel de silice.

La présente invention concerne le procédé de préparation du dérivé imidazoline de formule générale I, à partir du dérivé de formule générale F susceptible d'être obtenu par le procédé selon l'invention précédemment décrit.

Selon un mode préféré de réalisation de l'invention, le dérivé de formule générale IV est transformé en un dérivé 2-carboxamide-2-éthyl-2,3-dihydrobenzofurane de formule générale III 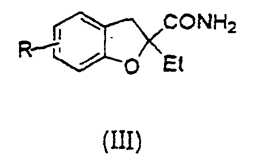 par une réaction de déshydroxylation, par exemple par hydrogénation catalytique.

Le dérivé de formule générale IV est dissous dans un solvant protique polaire anhydre, par exemple l'éthanol anhydre. On introduit ensuite dans le mélange un catalyseur d'hydrogénation, par exemple le palladium sur charbon activé. La réaction est conduite sous atmosphère d'hydrogène ; le mélange est vigoureusement agité, à une température comprise entre 10 et 30°C, pendant environ 20 heures. Après filtration, le dérivé carboxamide de formule générale III obtenu peut être purifié par des méthodes classiques, par exemple par chromatographie sur gel de silice.

Le dérivé carboxamide de formule générale III ainsi obtenu est transformé, par le procédé de préparation selon l'invention, en dérivé imidazoline de formule générale I en une ou deux étapes, i.e. en passant ou en ne passant pas par le dérivé 2-cyano-2-éthyl-2.3-dihydro-benzofurane de formule générale II

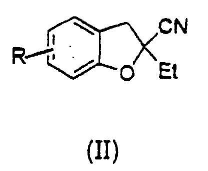

Sans passer par le dérivé nitrile de formule générale II, le dérivé carboxamide de formule générale III est traité par un agent d'O-alkylation de la fonction amide puis par l'éthylène diamine.

Le dérivé carboxamide de formule générale III est dissous dans un solvant polaire aprotique anhydre, par exemple choisi parmi le dichlorométhane, I'acétonitrile ou le diéthyl-éther, puis traité par un agent d'O-alkylation, par exemple un oxonium ou un sulfonium, en particulier le triéthyloxonium ou le diphényléthylsulfonium, sous atmosphère inerte, sous agitation, à une température comprise entre 10°C et la température d'ébullition du solvant, pendant 40 à 100 heures.

Le solvant est ensuite évaporé. Le résidu obtenu est repris avec un solvant polaire protique, de préférence I'éthanol puis traité par un excès d'éthylène diamine, sous agitation, à une température comprise entre 10 et 30°C pendant 40 à 70 heures.

Le dérivé imidazoline de formule générale I peut être purifié par des méthodes classiques, par exemple extraction par un solvant organique, de préférence le diéthyl-éther ou le dichlorométhane, puis chromatographie sur gel de silice.

En passant par le dérivé nitrile de formule générale II, le dérivé carboxamide de formule générale III est dissous dans un solvant polaire protique, par exemple le dioxane, puis traité par un anhydride d'acide, de préférence l'anhydride trifluoroacétique en présence d'une amine tertiaire, en particulier la triéthylamine, sous agitation, à une température comprise entre -2 et +5°C, pendant 1 à 2 heures.

Le milieu réactionel est ensuite hydrolysé par de l'eau glacée pour obtenir le dérivé nitrile de formule générale II, qui peut être purifié par extraction à l'aide d'un solvant organique, par exemple l'hexane.

Dans un autre mode de réalisation de l'invention, le dérivé nitrile de formule générale II est obtenu par déshydratation du dérivé carboxamide correspondant de formule générale III, en utilisant le chlorure de phosphoryle (POCl₃), le pentaoxyde de diphosphore (P₂O₅) ou le chlorure de thionyle (SOCl₂).

Le dérivé imidazoline de formule générale I est préparé selon l'invention en traitant le dérivé nitrile de formule générale II, obtenu par l'un quelconque des modes de réalisation précédents, par au moins un équivalent molaire d'éthylène diamine et un acide, de préférence l'acide chlorhydrique, dans un solvant polaire protique, par exemple un alcool en C₁ - C₄, en particulier l'éthanol ou le méthanol, à une température comprise entre 0 et 10°C.

Lorsque les étapes d'addition de l'éthylène diamine et de l'acide se font successivement, leur ordre peut être inversé. Les deux étapes peuvent aussi être simultanées.

Selon un mode de réalisation de l'invention, on ajoute l'acide chlorhydrique dissous dans l'alcool à la solution alcoolique de nitrile (que l'on peut préalablement traiter par un alkoxyde alcalin, par exemple le méthoxyde ou l'éthoxyde de sodium, à température ambiante). Puis, dans un second temps, on ajoute l'éthylène diamine à une température comprise entre 0 et 10°C.

Selon le mode de réalisation préféré de l'invention, on laisse agir l'éthylène diamine dans la solution alcoolique de nitrile saturée en acide chlorydrique, pendant 12 à 24 heures, à une température comprise entre 0 et 10°C.

Le dérivé imidazoline de formule générale I obtenu après addition de l'éthylène diamine et de l'acide chlorhydrique, selon l'un quelconque des modes de réalisation précédemment décrits, est purifié par extraction au solvant chloré, de préférence le dichlorométhane ou le trichlorométhane, puis désalifié ou non selon que l'on souhaite obtenir le dérivé imidazoline de formule générale I sous forme libre ou sous forme de sel.

La figure 1 représente le schéma réactionnel du procédé de préparation des dérivés 2-(2-(2-ethyl-2, 3-dihydrobenzofuranyl))-2-imidazoline selon l'invention.

D'autres caractéristiques du procédé selon l'invention apparaîtront à la lumière des exemples suivants.

### Exemple 1 : Synthèse du 2-éthylcarboxylate-2-éthyl-3-hydroxy-2,3-dihydrobenzofurane

Dans un bicol de 50 ml, muni d'un réfrigérant à reflux et préalablement dégazé, on introduit successivement 0,51 g d'aldéhyde salicylique (4,18 mmol ; 1 éq), 20 ml de diméthylformamide (DMF) anhydre, 0,74 ml de 2-bromobutanoate d'éthyle (5,02 mmol ; 1,2 éq), 1,271 g de carbonate de potassium (9,2 mmol ; 2,2 éq) et 0,125 g d'iodure de sodium (0,84mmol ; 0,2 éq). Le mélange (qui n'est pas homogène) est agité et porté au reflux du DMF pendant 9 heures. La solution noircit. Après refroidissement, le DMF est éliminé par évaporation à l'aide d'un montage dit "Hickmann" (vide : pompe à palette). Les dernières traces de DMF sont éliminées par extraction : on ajoute 10 ml d'eau et 1 ml d'HC1 concentré (la phase aqueuse doit se décolorer) et on extrait avec de l'acétate d'éthyle. Le brut réactionnel est purifié par chromatographie sur gel de silice (éluant : Hexane/Et₂O : 7/3. Révélateur : PMA). On obtient 0,77 g d'un mélange de deux diastéréoisomères, que l'on notera désormais A et B, A/B : 76/24, ce qui correspond à rendement global de 78 % à partir de l'aldéhyde salicylique.

La structure des diastéréoisomères A et B est déterminée par l'expérience NOESY.

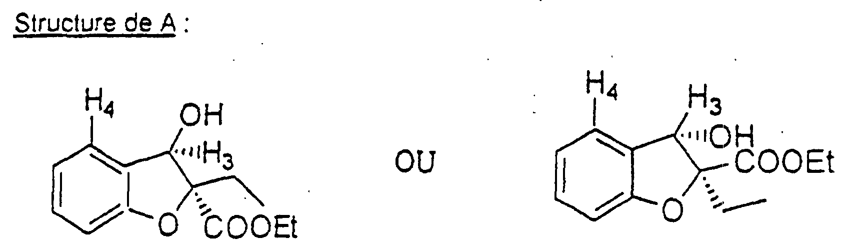

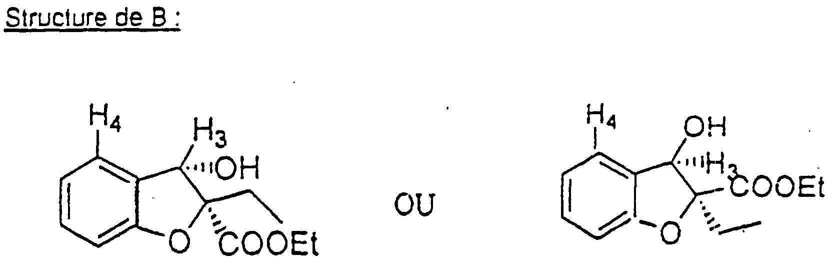

### Caractérisation de A :

Aspect : Huile jaune pâle.
RMN ¹H (CDCl₃ ; 200 MHz) : 7,40-7,25 (massif, 2H, Phényl) ; 7,0-6,92 (massif, 2H, Phényl) ; 5,35 (d, 1H, H₃, J=8,0 Hz); 4,26-4,13 (q.2H, COOCH₂CH₃) ; 2,28-1,98 (massif, 3H, CH₂CH₃ + OH) ; 1,26 (t, 3H, COOCH₂CH₃).
RMN ¹³C (CDCl₃; 50 MHz): 172,47 (COOEt) ; 158,84; 130,65; 127,10; 125,66; 121,30; 110,47; 93,84; 75,93; 61,64; (COOCH₂CH₃); 24,70 (CH₂CH₃); 13,99 (COOCH₂CH₃); 8,27 (CH₂CH₃).
Spectographie de masse (IC/NH₃): 254 (M+NH₄⁺); 236 (M⁺); 219; 163; 102; 88.

### Caractérisation de B :

Aspect : Huile jaune pâle.
RMN ¹H (CDCl₃; 200 MHz): 7,42-7,25 (massif, 2H, Phényl); 7,00-6,92 (massif, 2H, Phényl); 5,02 (d, 1H, H₃, J=7,3 Hz); 4,39-4,18 (q, 2H, COOCH₂CH₃); 2,58 (d, 1H, OH, J=7,3 Hz); 2,19-2,01 (m, 1H, CH₂CH₃); 1,82-1,64 (m, 1H, CH₂CH₃); 1,33 (t, 3H, COCCH₂CH₃); 0,99 (t, 3H, CH₂CH₃).
RMN ¹³C (CDCl₃; 50 MHz): 182,37 (COOEt); 169,84; 159,07; 131,14; 126,00; 121,50; 111,10; 95,08; 78,53; 61,78 (COOCH₂CH₃); 29,24 (CH₂CH₃); 14,31 (COOCH₂CH₃); 8,27 (CH₂CH₃).

### Exemple 2 : Synthèse du 2-carboxamide-2-éthyl-3-hydroxy-2,3-dihydrofurane

### Méthode 1:par l'ammoniaque

Un mélange des deux diastéréoisomères A et B (A/B: 76/24) est dissous dans 0,45 ml de dioxane. A cette solution (placée dans un vase clos), on ajoute 0,89 ml d'une solution ammoniaquale à 30 % (10 éq.). On bouche le vase clos et on agite à T (°C) pendant t (h). Puis on évapore le dioxane et on extrait au dichlorométhane. Le brut est purifié par chromatographie sur gel de silice (éluant: Et₂O/Hexane: 7/3. Révélateur: Vanilline). On obtient du réactif et un solide blanc qui correspond à l'amide formé.

| T(°C) | t(h) | réactif (%) | amide formé (%) |
|---|---|---|---|
| 20 | 140 | 45 | 32 |
| 50 | 66 | 21 | 36 |

### Méthode 2 : par l'ammoniac

Dans un bicol de 35 ml, on dissout 0,9 g d'un mélange des deux diatéréoisomères A et B, préparé selon l'exemple 1 (A/B; 46/54), dans 11 ml de méthanol anhydre. On sature la solution en ammoniac gazeux puis on bouche le ballon avec des septa et on laisse sous agitation magnétique à une température comprise entre 10 et 30°C pendant 5 jours. Le méthanol est ensuite évaporé et le mélange brut chromatographié sur gel de silice (éluant: Et₂O/Hexane: 7/3. Révélateur: Vanilline). L'amide obtenu est difficilement soluble dans le chloroforme, soluble dans le méthanol. Le rendement est de 62 % en considérant le mélange A₁/B₁ des deux diastéroisomères obtenus. En partant du diastéréoisomère A pur, on obtient 82 % de diastéréoisomère A₁.

### Caractérisation du diastéréoisomère A₁:

RMN ¹H (CDCl₃, 200,13 MHz): 7,44-7,27 (massif, 2H, Phényl); 7,05-6,89 (massif, 2H, Phényl); 6,60 (massif, 1H, CONH₂); 5,60 (massif, 1H, CONH₂); 5,50 (s, 1H, H₃); 2,57 (d, 1H, OH, J=6,5Hz); 2,28-1,91 (massif, 3H, CH₂-CH₃ + OH); 1,08 (t, 3H, CH₂-CH₃).
RMN ¹H (CD₃OD; 200,13 MHz): 7,38-6,85 (massif, 4H, Phényl); 5,29 (s, 1H, H₃); 2,18-1,86 (massif, 2H, CH2-CH₃); 1,07 (t, 3H, CH₂-CH₃).
Point de fusion: 148-149°C.

### Caractérisation du diastéréoisomère B₁:

RMN ¹H (CD₃OD; 200,13 MHz): 7,59-6,92 (massif, 4H Phényl); 4,95 (s, 1H, H₃); 2,20-1,93 (massif, 1H, CH₂-CH₃); 1,65-1,42 (massif, 1H, CH₂-CH₃); 0,96 (t, 3H, CH₂-CH₃).

### Exemple 3: Synthèse du 2-éthyl-2-carboxamide-2,3-dihydrobenzofurane

Dans un bicol de 25 ml, on dissout 0,167 g des deux diastéréoisomères AI et Bl (Al/Bl: 42/58) (0,807 mmol), obtenus selon l'exemple 2 dans 10 ml d'éthanol anhydre. Le ballon est dégazé à l'argon. On introduit alors une pointe de spatule de Palladium sur charbon activé à 10 %. On dégaze de nouveau, cette fois avec un peu d'hydrogène puis on se place sous atmosphère d'hydrogène et on agite vigoureusement à une température comprise entre 10 et 30°C pendant 20 heures sous pression atmosphérique. On filtre ensuite la solution sur celite (éluant: EtOH). Le brut réactionnel résultant est chromatographié sur gel de silice (éluant: Et₂O/Hexane: 7/3. Révélateur: Vanilline ou PMA). L'amide déshydroxylé est obtenu avec un rendement de 88 %.

En partant du diastéréoisomère A₁ pur, le rendement est de 93%.

### Caractérisation du 2-éthyl-2-carboxamide-2.3-dihydrofurane :

RMN ¹H (CDCl₃; 200,13 MHz): 7,18-6,81 (massif, 4H, Phényl); 6,69 (massif. 1H, CONH₂); 6,34 (massif, 1H, CONH₂); Système AB.&A=3,57, &B=3,17, JAB=16,44 Hz; 2,21-1,82 (massif, 2H, CH₂-CH₃); 1,01 (t, 3H, CH₂-CH₃).

### Exemple 4 : Synthèse 2-(2-(2-éthyl-2,3-dihydro-benzofuranyl))-2-imidazoline à partir du 2-éthyl-2-carboxamide-2,3-dihydrobenzofurane

### Avec le réactif de MEERWEIN : Et₃O+BF₄

A une solution de 0,2 g de 2-éthyl-2-carboxamide-2,3-dihydrobenzofurane (1,05 mmol) dans 5 ml de dichlorométhane anhydre, on ajoute doucement une solution de 0,23 g d'oxonium (1,21 mmol; 1,1 éq.) dans 3 ml de dichlorométhane sec. On agite sous atmosphère inerte à une température comprise entre 10 et 30°C pendant 66 heures. La solution est évaporée sous vide, la pâte obtenue est reprise dans 2 ml d'éthanol et 0,8 d'éthylène diamine (12 mmol; 11 éq) sont ajoutés. Le ballon est bouché puis la solution agitée à une température comprise entre 10 et 30°C pendant 47 heures. L'éthanol est évaporé; le brut est repris avec un peu d'eau et extrait à l'éther (3x10 ml). Le brut réactionnel est purifié par chromatographie sur gel de silice (éluant: : Hexane/Et₂O: 5/5. Révélateur : vanilline). Le rendement est de 72%.

### Avec le diphényléthyl sulfonium : Ph₂S+EtBF₄

0,155 g (0,81 mmol) de 2-éthyl-2-carboxamide-2,3-dihydrobenzofurane sont dissous dans 10 ml d'acétonitrile. On ajoute 0,49 g de sulfonium (1,62 mmol; 2 éq) et on porte le mélange au reflux. Bien qu'après 66 heures le mélange ne semble plus évoluer (suivi ccm), on prolonge le reflux jusqu'à 100 heures car il reste du réactif. On évapore alors I'acétonitrile et on reprend le mélange avec 2 ml d'éthanol avant d'ajouter l'éthylène diamine en excès (10éq). On agite à une température comprise entre 10 et 30°C pendant 69 heures. On évapore ensuite l'éthanol, on reprend avec un peu d'eau et on extrait au dichlorométhane (3x20 ml). Une filtration rapide sur colonne (éluant Et₂O/Hexane : 7/3) permet d'éliminer le diphénylsulfure formé et le réactif. Le rendement est de 46 %.

### Caractérisation du 2-(2-(2-éthyl-2.3-dihydro-benzofuranyl)):

RMN ¹H (CDCl₃; 200.13 MHz): 7.27-6.79 (massif, 4H, Phényl); 4.50 (massif, 1H, NH); entre 3.70 et 3.57, un massif correspondant à une partie d'AB et aux 4H de l'imidazole; à 3.20, l'autre partie de l'AB, J_{AB} = 16.1 Hz; 2.13-1.85 (massif, 2H, CH₂-CH3); 0.97 (t, 3H, CH₂-CH₃).

### Exemple 5 : Synthèse du 2-(2-(2-éthyl-2,3-dihydro-benzofurany))-2-imidazoline à partir du 2-cyano-2-éthyl-2,3-dihydrobenzofurane

### A) Préparation du 2-cyano-2-éthyl-2,3-dihydrobenzofurane.

Dans un réacteur de 2L muni d'une agitation mécanique et d'une ampoule à addition sont introduits, (m=120 g - 0.628 moles) dilués dans 360 ml de dioxane et la triéthylamine (V = 184 ml - 1.32 moles). Le milieu réactionnel est refroidi à -2°/0°C. L'anhydride trifluoroacétique (V=106.8 ml - 0.756 moles) est additionné en 45 mn, en maintenant la température entre 0°C et + 5°C par refroidissement. Le milieu réactionnel est agité à cette température pendant 45mn, hydrolysé avec 480 ml d'eau glacée, et agité 15 mn en laissant la température revenir à 20°C environ.

Le composé nitrile est extrait avec 330 ml puis 100 ml d'hexane. Les phases hexaniques rassemblées sont lavées avec 360 ml de solution aqueuse à 10% d'hydrogénocarbonate de sodium, puis neutralisées et débarrassées du dioxane résiduel par lavage aqueux. Après séchage sur sulfate de sodium et concentration, le 2-cyano-2-éthyl-2,3-dihydrobenzofurane (m = 104,7 g) est obtenu avec un rendement de 96%.

### B) Préparation de l'Efaroxan : 2-(2-(2-éthyl-2,3-dihydrobenzofurany))-2-imidazoline.

Dans un tricol de 500 ml équipé pour le barbotage d'acide chlorhydrique, le 2-cyano-2-éthyl-2,3-dihydrobenzofurane obtenu selon l'exemple 5.A (m=63.3 g - 0.365 moles) est introduit dilué dans 250 ml d'éthanol 100. Le milieu est refroidi à 0°C, température à laquelle on débute le barbotage d'acide chlorhydrique régulé de manière à maintenir la température entre 0°C et 10°C, et ce jusqu'à saturation de la solution éthanolique (environ 3 heures). La réaction est suivie par chromatographie sur couche mince. Le milieu réactionnel est refroidi entre 0°C et 10°C et l'éthylènediamine (36.7 ml - 0.55 moles) diluée dans 30 ml d'éthanol 100 est additionnée lentement à une température inférieure à 10°C. Le précipité de dichlorhydrate d'éthylène diamine obtenu est filtré et quantifié après extrait sec. On en déduit la quantité d'éthylènediamine (5 ml) qu'il faut ajouter pour arriver à un excès de 10% par rapport au nitrile. Le milieu réactionnel est agité pendant 12 à 24 heures à environ 20°C. Le milieu réactionnel est salifié par une solution à 5.7 N d'isopropanol/acide chlorhydrique (115 ml). L'excès d'éthylène diamine est éliminé sous forme de dichlorhydrate par filtration et le chlorhydrate d'Efaroxan est repris dans 1L d'eau après évaporation des solvants. La phase aqueuse est lavée avec 2x300 ml de dichlorométhane. Le chlorhydrate d'Efaroxan est désalifié par 70 ml d'ammoniaque 27% en Efaroxan que l'on extrait par 300 ml de dichlorométhane. La phase organique est lavée avec 400 ml d'eau, séchée sur sulfate de sodium et concentrée à l'évaporateur rotatif. L'Efaroxan (m=71.6 g) est obtenu avec un rendement de 90%.

## Revendications

1. Dérivé du 2-éthyl-3-hydroxy-2,3-dihydro-benzofurane de formule générale F 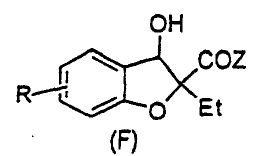 dans laquelle R représente un atome d'hydrogène, un halogène, un radical alkyle en C₁-C₃, alkoxy en C₁-C₃ du hydroxy et Z représente un groupement alkoxy en C₁-C₃, ou un groupement amino,
à l'exception toutefois du dérivé de formule générale F dans laquelle R représente un atome d'hydrogène et Z représente le groupement éthoxy.

2. Procédé de préparation du dérivé du 2-éthyl-3-hydroxy-2,3-dihydrobenzofurane de formule générale F indiquée dans la revendication 1 dans laquelle R représente un atome d'hydrogène, un halogène, un radical alkyle inférieur, alkoxy inférieur ou hydroxy et Z représente un groupement alkoxy en C₁-C₃. de préférence éthoxy, ou un groupement amino, **caractérisé en ce que** le dérivé de formule générale F est obtenu à partir du dérivé d'aldéhyde salicylique correspondant.

3. Procédé selon la revendication 2, **caractérisé en ce qu'**un équivalent de dérivé d'aldéhyde salicylique est traité par un équivalent de 2-bromobutanoate d'éthyle, deux équivalents de base; par exemple le carbonate de potassium, dans un solvant polaire aprotique anhydre, en particulier le diméthylformamide, en présence d'un catalyseur, en particulier l'iodure de sodium, pour obtenir un dérivé d'ester.

4. Procédé selon l'une quelconque des revendications 2 et 3, **caractérisé en ce que** le dérivé d'ester subit une aminolyse.

5. Procédé selon la revendication 4, **caractérisé en ce que** l'aminolyse est effectuée par une solution d'hydroxyde d'ammonium, dans un solvant polaire aprotique, par exemple le dioxane.

6. Procédé selon la revendication 4, **caractérisé en ce que** l'aminolyse est effectuée dans un solvant polaire protique, par exemple un alcool aliphatique en C₁-C₃, de préférence le méthanol, saturé en ammoniac

7. Procédé de préparation du 2-[2-(2-éthyl-2,3-dihydrobenzofuranyl]-2-imidazoline et de ses analogues de formule générale I 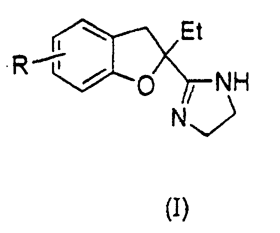 dans laquelle R représente un atome d'hydrogène, un halogène, un radical alkyle inférieur, alkoxy inférieur ou hydroxy, **caractérisé en ce que** l'on utilise un dérivé de formule générale F selon la revendication 1 susceptible d'être obtenu par le procédé selon l'une des revendications 2 à 6.

8. Procédé selon la revendication 7, **caractérisé en ce que** le composé de formule générale I est obtenu en traitant le dérivé du 2-carboxamide-2-éthyl-3-hydroxy-2,3-dihydro-benzofurane de formule générale IV 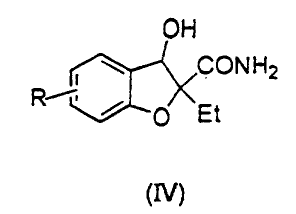 dans laquelle R représente un atome d'hydrogène, un halogène, un radical alkyle inférieur, alkoxy inférieur ou hydroxy, par une réaction de deshydroxylation, par exemple par hydrogénation catalytique pour obtenir un dérivé de formule générale III 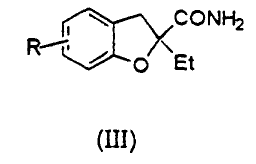 dans laquelle R représente un atome d'hydrogène, un halogène, un radical alkyle inférieur, alkoxy inférieur ou hydroxy.

9. Procédé selon l'une quelconque des revendications 7 et 8, **caractérisé en ce que** le composé de formule générale IV, subit une deshydroxylation catalysée par le palladium sur charbon activé sous atmosphère d'hydrogène en milieu polaire anhydre, pour obtenir un dérivé de formule générale III 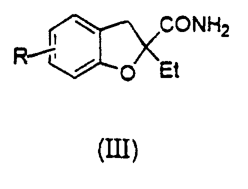 dans laquelle R représente un atome d'hydrogène, un radical alkyle inférieur, alkoxy inférieur ou hydroxy.

10. Procédé selon l'une des revendications 8 ou 9, **caractérisé en ce que** le composé de formule générale III est traité par un agent d'O-alkylation de la fonction amide puis par l'éthylène diamine.

11. Procédé selon la revendication 10, **caractérisé en ce que** l'agent d'O-alkylation est un oxonium, en particulier le triéthyloxonium, ou un sulfonium, en particulier le diphénylsulfonium.

12. Procédé selon l'une des revendications 8 ou 9, **caractérisé en ce que** le composé de formule générale III est traité par un anhydre d'acide, de préférence l'anhydre trifluoroacétique, en présence d'une amine tertiaire, en particulier la triéthylamine, dans un solvant polaire protique, pour obtenir un dérivé nitrile.

13. Procédé selon la revendication 12, **caractérisé en ce que** le dérivé nitrile est dissous dans un alcool en C₁ - C₄, de préférence l'éthanol ou le méthanol, puis traité par l'éthylène diamine et un acide, en particulier l'acide chlorhydrique.

## Patentansprüche

1. Derivat von 2-Ethyl-3-hydroxy-2,3-dihydrobenzofuran der allgemeinen Formel F 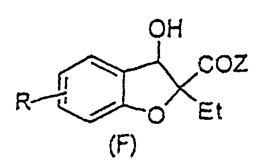 in der R ein Wasserstoffatom, ein Halogen, einen C₁-C₃-Alkylrest, einen C₁-C₃-Alkoxyrest oder Hydroxy darstellt und Z eine C₁-C₃-Alkoxygruppe oder eine Aminogruppe darstellt,
jedoch mit der Ausnahme des Derivates der allgemeinen Formel F, in der R ein Wasserstoffatom und Z eine Ethoxygruppe darstellt.

2. Verfahren zur Herstellung des Derivates von 2-Ethyl-3-hydroxy-2,3-dihydrobenzofuran der allgemeinen Formel F nach Anspruch 1, in der R ein Wasserstoffatom, ein Halogen, einen niederen Alkylrest, einen niederen Alkoxyrest oder Hydroxy darstellt und Z eine C₁-C₃-Alkoxygruppe, vorzugsweise Ethoxy, oder eine Aminogruppe darstellt, **dadurch gekennzeichnet, dass** das Derivat der allgemeinen Formel F ausgehend von dem entsprechenden Salicylaldehyd-Derivat erhalten wird.

3. Verfahren nach Anspruch 2, **dadurch gekennzeichnet, dass** ein Äquivalent des Salicylaldehyd-Derivates mit einem Äquivalent Ethyl-2-Brornbutanoat, zwei Äquivalenten Base, z.B. Kaliumcarbonat, in einem polaren aprotischen wasserfreien Lösungsmittel, insbesondere Dimethylformamid, in Anwesenheit eines Katalysators, insbesondere Natriumiodid, behandelt wird, um ein Esterderivat zu erhalten.

4. Verfahren nach einem der Ansprüche 2 und 3, **dadurch gekennzeichnet, dass** das Esterderivat einer Aminolyse unterworfen wird.

5. Verfahren nach Anspruch 4, **dadurch gekennzeichnet, dass** die Aminolyse mit einer Ammoniumhydroxid-Lösung in einem polaren aprotischen Lösungsmittel, z.B. Dioxan, durchgeführt wird.

6. Verfahren nach Anspruch 4, **dadurch gekennzeichnet, dass** die Aminolyse in einem polaren protischen Lösungsmittel, z.B. in einem mit Ammoniak gesättigten aliphatischen C₁-C₃-Alkohol, vorzugsweise Methanol, durchgeführt wird.

7. Verfahren zur Herstellung von 2-[2-(2-Ethyl-2,3-dihydrobenzofuranyl]-2-imidazolin und Analoga davon der allgemeinen Formel I 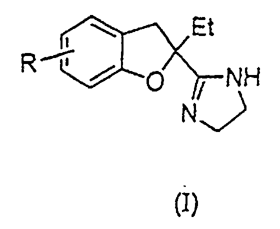 in der R ein Wasserstoffatom, ein Halogen, einen niederen Alkylrest, einen niederen Alkoxyrest oder Hydroxy darstellt, **dadurch gekennzeichnet, dass** man ein Derivat der allgemeinen Formel F nach Anspruch 1 verwendet, erhältlich durch das Verfahren nach einem der Ansprüche 2 bis 6.

8. Verfahren nach Anspruch 7, **dadurch gekennzeichnet, dass** die Verbindung der allgemeinen Formel I dadurch erhältlich ist, dass das Derivat von 2-Carboxamid-2-ethyl-3-hydroxy-2,3-dihydrobenzofuran der allgemeinen Formel IV 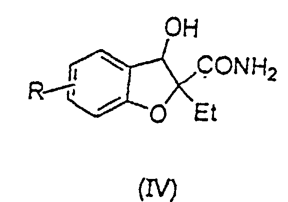 in der R ein Wasserstoffatom, ein Halogen, einen niederen Alkylrest, einen niederen Alkoxyrest oder Hydroxy darstellt, einer Dehydroxylierungs-Reaktion unterworfen wird, z.B. einer katalytischen Hydrierung, um ein Derivat der allgemeinen Formel III zu erhalten 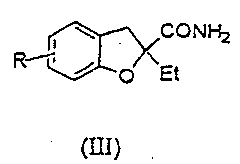 in der R ein Wasserstoffatom, ein Halogen, einen niederen Alkylrest, einen niederen Alkoxyrest oder Hydroxy darstellt.

9. Verfahren nach einem der Ansprüche 7 und 8, **dadurch gekennzeichnet, dass** die Verbindung der allgemeinen Formel IV einer durch Palladium auf aktiviertem Kohlenstoff katalysierten Dehydroxylierung in Wasserstoffatmosphäre in wasserfreiem polarem Milieu unterworfen wird, um ein Derivat der allgemeinen Formel III zu erhalten 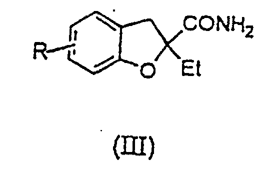 in der R ein Wasserstoffatom, einen niederen Alkylrest, einen niederen Alkoxyrest oder Hydroxy darstellt.

10. Verfahren nach einem der Ansprüche 8 oder 9, **dadurch gekennzeichnet, dass** die Verbindung der allgemeinen Formel III mit einem O-Alkylierungsmittel der Amid-Funktion und dann mit Ethylendiamin behandelt wird.

11. Verfahren nach Anspruch 10, **dadurch gekennzeichnet, dass** das O-Alkylierungsmittel ein Oxonium, insbesondere Triethyloxonium, oder ein Sulfonium, insbesondere Diphenylsulfonium, ist.

12. Verfahren nach einem der Ansprüche 8 oder 9, **dadurch gekennzeichnet, dass** die Verbindung der allgemeinen Formel III mit einem Säureanhydrid, vorzugsweise Trifluoressigsäureanhydrid, in Gegenwart eines tertiären Amins, insbesondere Triethylamin, in einem polaren protischen Lösungsmittel behandelt wird, um ein Nitrilderivat zu erhalten.

13. Verfahren nach Anspruch 12, **dadurch gekennzeichnet, dass** das Nitrilderivat in einem C₁-C₄-Alkohol, vorzugsweise Ethanol oder Methanol, gelöst und dann mit Ethylendiamin und einer Säure, insbesondere Chlorwasserstoffsäure, behandelt wird.

## Claims

1. 2-Ethyl-3-hydroxy-2,3-dihydrobenzofuran compound of general formula F 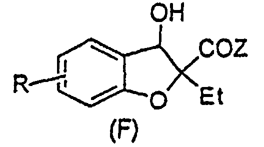 wherein R represents a hydrogen atom, a halogen, a C₁-C₃-alkyl radical, a C₁-C₃-alkoxy radical or hydroxy and Z represents a C₁-C₃-alkoxy group or an amino group, with the exception, however, of the compound of general formula F wherein R represents a hydrogen atom and Z represents the ethoxy group.

2. Process for the preparation of the 2-ethyl-3-hydroxy-2,3-dihydrobenzofuran compound of general formula F indicated in claim 1 wherein R represents a hydrogen atom, a halogen, a lower alkyl radical, a lower alkoxy radical or hydroxy and Z represents a C₁-C₃-alkoxy group, preferably ethoxy, or an amino group, **characterised in that** the compound of general formula F is obtained starting from the corresponding salicylic aldehyde compound.

3. Process according to claim 2, **characterised in that** one equivalent of salicylic aldehyde compound is treated with one equivalent of ethyl 2-bromobutanoate and two equivalents of base, for example potassium carbonate, in an anhydrous aprotic polar solvent, especially dimethylformamide, in the presence of a catalyst, especially sodium iodide, to obtain an ester compound.

4. Process according to any one of claims 2 and 3, **characterised in that** the ester compound is subjected to aminolysis.

5. Process according to claim 4, **characterised in that** the aminolysis is carried out by means of a solution of ammonium hydroxide in an aprotic polar solvent, for example dioxane.

6. Process according to claim 4, **characterised in that** the aminolysis is carried out in a protic polar solvent, for example an aliphatic C₁-C₃-alcohol, preferably methanol, saturated with ammonia.

7. Process for the preparation of 2-[2-(2-ethyl-2,3-dihydrobenzofuranyl)]-2-imidazoline and its analogues of general formula I 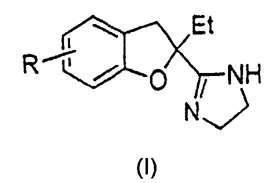 wherein R represents a hydrogen atom, a halogen, a lower alkyl radical, a lower alkoxy radical or hydroxy, **characterised in that** there is used a compound of general formula F according to claim 1 obtainable by the process according to one of claims 2 to 6.

8. Process according to claim 7, **characterised in that** the compound of general formula I is obtained by treating the 2-carboxamide-2-ethyl-3-hydroxy-2,3-dihydrobenzofuran compound of general formula IV 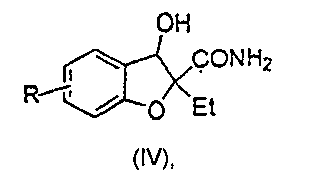 wherein R represents a hydrogen atom, a halogen, a lower alkyl radical, a lower alkoxy radical or hydroxy, by means of a dehydroxylation reaction, for example by means of catalytic hydrogenation, to obtain a compound of general formula III 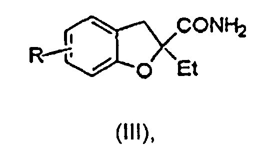 wherein R represents a hydrogen atom, a halogen, a lower alkyl radical, a lower alkoxy radical or hydroxy.

9. Process according to any one of claims 7 and 8, **characterised in that** the compound of general formula IV is subjected to dehydroxylation catalysed by palladium on activated carbon under a hydrogen atmosphere in an anhydrous polar medium to obtain a compound of general formula III 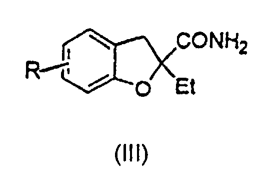 wherein R represents a hydrogen atom, a lower alkyl radical, a lower alkoxy radical or hydroxy.

10. Process according to one of claims 8 or 9, **characterised in that** the compound of general formula III is treated with an agent for O-alkylation of the amide function and then with ethylenediamine.

11. Process according to claim 10, **characterised in that** the agent for O-alkylation is an oxonium, especially triethyloxonium, or a sulphonium, especially diphenylsulphonium.

12. Process according to one of claims 8 or 9, **characterised in that** the compound of general formula III is treated with an acid anhydride, preferably trifluoroacetic anhydride, in the presence of a tertiary amine, especially triethylamine, in a protic polar solvent, to obtain a nitrile compound.

13. Process according to claim 12, **characterised in that** the nitrile compound is dissolved in a C₁-C₄-alcohol, preferably ethanol or methanol, and then treated with ethylenediamine and an acid, especially hydrochloric acid.
